Europäisches Patentamt

European Patent Office

Office européen des brevets

(19)

(11) **EP 0 709 404 B1**

(12) # EUROPÄISCHE PATENTSCHRIFT

(45) Veröffentlichungstag und Bekanntmachung des Hinweises auf die Patenterteilung:
**07.01.1998 Patentblatt 1998/02**

(51) Int Cl.6: **C08F 8/50**, C08F 10/00

(21) Anmeldenummer: **95116070.4**

(22) Anmeldetag: **12.10.1995**

(54) **Verfahren zum thermomechanischen Abbau von Polyolefinen**

Process for thermomechanical degradation of polyolefins

Procédé de dégradation thermomécanique de polyoléfines

(84) Benannte Vertragsstaaten:
**AT BE DE ES FR GB IT NL**

(30) Priorität: **21.10.1994 DE 4437754**

(43) Veröffentlichungstag der Anmeldung:
**01.05.1996 Patentblatt 1996/18**

(73) Patentinhaber: **BASF AKTIENGESELLSCHAFT**
**67056 Ludwigshafen (DE)**

(72) Erfinder:
• **Deckers, Andreas, Dr.**
**D-55234 Flomborn (DE)**
• **Berger, Albin, Dr.**
**D-67273 Bobenheim (DE)**
• **Hofmann, Jürgen**
**D-67069 Ludwigshafen (DE)**
• **Klimesch, Roger, Dr.**
**D-64665 Alsbach-Hähnlein (DE)**
• **Farwerck, Karl-Peter**
**D-67551 Worms (DE)**
• **Ohlig, Hilmar**
**D-67657 Kaiserslautern (DE)**

(56) Entgegenhaltungen:
EP-A- 0 349 828        EP-A- 0 474 889
DE-A- 1 520 505        DE-A- 3 225 875
US-A- 4 001 172        US-A- 4 578 430

**Beschreibung**

Die vorliegende Erfindung betrifft ein Verfahren zum thermomechanischen Abbau von Polyolefinen in einem Zweiwellenextruder ohne nachgeschalteter Abbauapparatur, bei Temperaturen von 300 bis 550°C, Drücken von 1 bis 100 bar und mittleren Verweilzeiten des Reaktionsgemisches von 0,5 bis 10 Minuten.

Die Herstellung von Polyolefinwachsen, insbesondere Polypropylenwachsen in einem breiten Molekulargewichtsbereich bei gleichzeitig enger Molekulargewichtsverteilung durch eine kontrollierte Polymerisation nach einem wirtschaftlichen Verfahren ist bisher nicht möglich. Derartige Wachse werden daher überwiegend durch thermischen Abbau aus hochmolekularen Polyolefinen gewonnen (US-A 3 087 922, FR-A 1 289 767, FR-A 1 252 635, FR-A 1 425 695, US-A 3 519 609, US-A 3 562 788, CA-A 797 293, DD-A 50 113).

Aus der DE-C 3 003 768 ist ein Verfahren zur Herstellung von Polyolefinwachs bekannt, bei welchem ein Gemisch aus Polyethylen und Polypropylen innerhalb eines beheizten Reaktors, insbesondere einem beheizten Metallrohr thermisch abgebaut wird. Dabei ist das beheizte Metallrohr in zwei unterschiedliche Zonen eingeteilt, die sich dadurch unterscheiden, daß sich in ihnen die Reaktionsmedien einmal im homogenen und zum anderen im heterogenen Zustand befinden. Bei diesem Verfahren wird die zum thermischen Abbau benötigte Wärmeenergie in der Weise dem Reaktionsmedium hinzugefügt, daß beide Zonen jeweils unterschiedlich stark beheizt werden.

Weiterhin wird in der DE-B 1 940 686 ein Verfahren zum kontinuierlichen Herstellen von wachsartigem, niedermolekularem Polyethylen aus festem hochmolekularem Polyethylen beschrieben, wobei das in einem Extruder aufgeschmolzene hochmolekulare Polyethylen in ein beheiztes Rohr übergeführt wird, wo es thermisch abgebaut wird. In dieser Patentschrift wird u.a. erwähnt, daß am Ende des beheizten Rohres durch ein Ventil oder eine Düse noch ein zusätzlicher Druck eingestellt werden kann, gegen den das Polyethylen extrudiert wird.

Aus der EP-A 0 474 889 ist ebenfalls ein Verfahren zum thermischen Zersetzen von Polymeren, insbesondere von Polyolefinen bekannt, nach welchem zunächst das Polymere in einem Extruder aufgeschmolzen wird und anschließend das dadurch erhältliche geschmolzene Polymere in einem Rohrreaktor einer thermischen Zersetzung unterworfen wird. Der dabei verwendete Rohrreaktor kann auch mit einem statischen Mischer versehen sein, um auf diese Weise eine effektivere Zersetzung des Polymeren zu erzielen.

In der Literatur ist darüber hinaus ein Verfahren zum thermomechanischen Abbau von Polyisobutylen/Polystyrol-Blends beschrieben [F.P. La Mantia, M.A. Nocilla in Polymer Degradation and Stability 17, 279-286 (1987)]. Aus dieser Publikation geht insbesondere hervor, daß der Abbau von Kunststoffblends sowohl durch Zufuhr von Wärmeenergie als auch durch Zufuhr von mechanischer Energie beeinflußt werden kann.

Die nach diesem Verfahren erhaltenen Wachse weisen jedoch häufig eine Reihe von Nachteilen auf, beispielsweise Gelbfärbung im Endprodukt oder hohe Anteile von Nebenprodukten, zum Beispiel Rußpartikel. Darüber hinaus wird bei diesem Verfahren insbesondere im Dauerbetrieb oft die Bildung von Wandbelägen und Anbackungen beobachtet. Bei der Herstellung von sehr niedermolekularen Polyolefinwachsen müssen außerdem in der Regel sehr lange Verweilzeiten in Kauf genommen werden, was mit erhöhten Kosten verbunden ist. Außerdem führt eine steigende Verweilzeit, d.h. ein steigender Abbaugrad zu einer steigenden Verfärbung. Die bisher bekannten Verfahren sind darüber hinaus oft nicht exakt steuerbar und führen insbesondere beim Abbau von Polypropylen zu erhöhten Anteilen an Rußpartikeln. Die dadurch erhaltenen niedermolekularen Polypropylene enthalten häufig zahlreiche, meist aperiodisch verteilte Rußpartikel, die für die spätere Anwendung, beispielsweise in sogenannten "Masterbatches" nachteilig sind.

Der vorliegenden Erfindung lag daher die Aufgabe zugrunde, ein neues Verfahren zu entwickeln, mit welchem der Abbau von Polyolefinen unter Vermeidung der bekannten Nachteile möglich wird und welches sich durch eine exakte Steuerbarkeit der Abbaureaktion und durch eine minimierte Bildung von Nebenprodukten, insbesondere von Rußpartikeln, auszeichnet.

Diese Aufgabe wurde gelöst durch ein Verfahren zum thermomechanischen Abbau von Polyolefinen in einem Zweiwellenextruder ohne nachgeschalteter Abbauapparatur, bei Temperaturen von 300 bis 550°C, Drücken von 1 bis 100 bar und mittleren Verweilzeiten des Reaktionsgemisches von 0,5 bis 10 Minuten, welches dadurch gekennzeichnet ist, daß der Druck im Zweiwellenextruder periodisch oder aperiodisch mit einer Schwankungsbreite von 0,5 bis 30 bar verändert wird.

Nach dem erfindungsgemäßen Verfahren wird der Abbau von Polyolefinen thermomechanisch durchgeführt, was bedeutet, daß die zur Spaltung der Polymerketten benötigten Energien sowohl in Form von thermischer Energie, als auch in Form von mechanischer Energie den Polyolefinen zugeführt werden. Der thermomechanische Abbau wird dabei in einem Zweiwellenextruder ohne nachgeschalteter Abbauapparatur, beispielsweise einem beheizten Rohr, vorgenommen. Als Zweiwellenextruder eignen sich dabei die in der Kunststofftechnik üblichen Zweiwellenextruder, die u.a. von den Firmen Werner & Pfleiderer, Berstorff, Leistritz, JSW oder Toshiba hergestellt werden.

Der thermomechanische Abbau erfolgt dabei bei Temperaturen von 300 bis 550°C, insbesondere von 350 bis 500°C, Drücken von 1 bis 100 bar, insbesondere von 5 bis 50 bar und mittleren Verweilzeiten des Reaktionsgemisches von 0,5 bis 10 Minuten, insbesondere von 0,5 bis 6 Minuten. Nach einer besonders bevorzugten Variante des erfindungsgemäßen Verfahrens erfolgt der thermomechanische Abbau bei 370 bis 470°C und mittleren Verweilzeiten von 0,5 bis 4 Minuten.

Das erfindungsgemäße Verfahren ist dabei dadurch gekennzeichnet, daß der Druck im Zweiwellenextruder periodisch oder aperiodisch mit einer Schwankungsbreite von 0,5 bis 50 bar verändert wird. Vorteilhafterweise wird beim erfindungsgemäßen Verfahren der Druck im Zweiwellenextruder periodisch oder aperiodisch mit einer Schwankungsbreite von 0,5 bis 10 bar, insbesondere mit einer Schwankungsbreite von 1 bis 5 bar verändert.

Es empfiehlt sich, das erfindungsgemäße Verfahren in der Weise auszuführen, daß der Abstand zwischen zwei Druckstößen im Bereich von 1 bis 2000 Sekunden, insbesondere im Bereich von 30 und 500 Sekunden liegt. Die einzelnen Druckstöße werden dabei u.a. durch eine Steuervorrichtung oder durch ein Druckhalteventil ausgelöst, welches mit einer Zeitschaltung versehen ist oder aber auch durch eine geringfügige Änderung des Durchsatzes. Durch die periodisch oder aperiodisch ausgelösten Druckstöße entstehen im Extruder Druckveranderungen, die insbesondere Belagbildungen an den Extruderwänden beseitigen. Vorzugsweise werden die Druckveränderungen periodisch durchgeführt.

Das erfindungsgemäße Verfahren wird insbesondere in der Weise durchgeführt, daß 15 bis 70 % der zum Abbau der Polyolefine benötigten Energie in Form von mechanischer Energie in den Zweiwellenextruder eingebracht werden. Vorteilhafterweise werden beim erfindungsgemäßen Verfahren 20 bis 60 % und insbesondere 25 bis 60 % der zum Abbau der Polyolefine benötigten Energie in Form von mechanischer Energie den Polyolefinen zugeführt. Die Zufuhr von mechanischer Energie kann dabei insbesondere durch die Drehzahlen der Extruderwellen gesteuert werden. Üblicherweise betragen diese etwa 100 bis 500 Umdrehungen pro Minute, insbesondere 200 bis 350 Umdrehungen pro Minute.

Nach dem erfindungsgemaßen Verfahren kann es sich empfehlen, den zu verwendenden Zweiwellenextruder in zwei unterschiedliche Zonen, eine Aufschmelzzone (I) und eine sich daran anschließende Zone mit homogener Phase (II) aufzuteilen. Die Aufteilung des Zweiwellenextruders erfolgt üblicherweise durch Scherelemente, beispielsweise Knetblöcke. Vorzugsweise wird in der zweiten Zone mit homogener Phase (II) noch ein zusätzlicher Druck von 1 bis 50 bar, insbesondere von 1 bis 30 bar angelegt. Bei diesem Druck handelt es sich dabei um den Druck, der durch entsprechende Ventile oder Düsen am Austrag des Extruders angelegt wird. Der zusätzliche Druck ist dabei von demjenigen Druck zu unterscheiden, der durch die Reaktanden und insbesondere durch deren Strömungswiderstand im System aufgebaut wird. Durch das Anlegen des zusätzlichen Drucks wird u.a. die Schaumbildung der bei der Abbaureaktion entstehenden flüchtigen Produkte unterdrückt und die Wärmeleitfähigkeit des Reaktionsgemisches verbessert. Dies wirkt sich positiv auf die Qualitat der entstehenden niedermolekularen Polyolefinwachse aus.

Die für den Abbau der Polyolefine verwendeten Zweiwellenextruder sind zur optimalen Verfahrensführung im allgemeinen in wenigstens zwei Zonen aufteilbar. In den einzelnen Zonen können dabei unterschiedliche Druck- und Temperaturbedingungen eingestellt werden. Die im Inneren des Zweiwellenextruders sind befindlichen Schnekkenkombinationen sind gleichsinnig oder gegensinnig drehend, kämmend und in ihrer Gestaltung den jeweiligen Arbeitsbedingungen in den einzelnen Abschnitten des Extruders angepaßt. Die Wahl der jeweils geeigneten Schnecken oder Schneckenelemente, die Drehzahlen sowie die Geometrie des Zweiwellenextruders, d.h. sein Längen/Durchmesser-Verhaltnis, hängen u.a. von den jeweiligen Förderbedingungen und den jeweils eingesetzten Polyolefinen ab und sind dem Fachmann aufgrund seines Fachwissens geläufig.

Vorzugsweise werden beim erfindungsgemaßen Verfahren die für den Abbau bestimmten Polyolefine zunächst über eine Einzugsöffnung in eine Aufschmelzzone (I) gebracht und von dort in Form einer Schmelze in eine Zone mit homogener Phase (II) übergeführt, wobei die Abbaureaktion bereits in der Aufschmelzzone einsetzt. Die dadurch erhältlichen niedermolekularen Polyolefine werden anschließend über die Austragsöffnung aus dem Zweiwellenextruder transportiert und dort aufgearbeitet, d.h. abgekühlt, entgast und konfektioniert.

Das erfindungsgemäße Verfahren läßt sich vorteilhaft in der Weise ausgestalten, daß 80 % der in den Zweiwellenextruder eingebrachten mechanischen Energie in der Aufschmelzzone (I) zugeführt wird. Nach einem besonders bevorzugten Verfahren werden sogar wenigstens 90 % der in den Zweiwellenextruder eingebrachten mechanischen Energie bereits in der Aufschmelzzone (I) zugeführt. Auf diese Weise erreicht man u.a. eine hohe Rückvermischung der Polyolefine in der Aufschmelzzone (I) sowie eine hohe Dissipation.

Die Zone mit homogener Phase (II) zeichnet sich insbesondere durch eine hohe axiale Durchmischung und eine geringe Rückvermischung der Schmelze aus. In der Zone mit homogener Phase (II) kann der endgültige Grad des Polyolefinabbaus durch die Temperaturführung und den Druck exakt gesteuert werden.

Die Aufteilung in eine Aufschmelzzone (I) und eine Zone mit homogener Phase (II) kann nach dem erfindungsgemäßen Verfahren sowohl in einem einzigen Zweiwellenextruder als auch in zwei oder mehreren hintereinander geschalteten Zweiwellenextrudern verwirklicht werden. Bevorzugt wird der Abbau der Polyolefine in einem einzigen Zweiwellenextruder durchgeführt.

Das erfindungsgemäße Verfahren eignet sich insbesondere zum thermomechanischen Abbau von Polyolefinen, Unter der Bezeichnung Polyolefine sollen in diesem Zusammenhang insbesondere Homopolymerisate des Ethylens, des Propylens sowie des Isobutylens verstanden werden. Weiterhin fallen unter diese Bezeichnung Copolymerisate dieser Monomeren mit anderen Comonomeren, beispielsweise mit $C_4$-$C_8$-$\alpha$-Olefinen, wie But-1-en, Pent-1-en oder Hex-1-en, sowie mit vinylaromatischen Comonomeren, beispielsweise mit Styrol. Selbstverständlich können als Polyolefine auch Copolymere des Propylens mit Ethylen und/oder weiteren $C_4$-$C_8$-$\alpha$-Olefinen, beispielsweise But-1-en verwendet werden. Es können auch Recyclate dieser Polyolefine und deren Mischungen mit anderen Kunststoffen abgebaut werden.

Homo- und Copolymerisate des Ethylens und des Propylens sind dem Fachmann bekannt und in der Literatur beschrieben, so daß bezüglich detaillierter Angaben zur Herstellung bzw. zur Zusammensetzung auf die betreffenden Literaturstellen verwiesen werden kann. Derartige Polymerisate sind üblicherweise durch Ziegler-Natta-Polymerisation, durch Polymerisation mit chromhaltigen Phillipskatalysatoren, durch Hochdruckpolymerisation in Anwesenheit radikalisch zerfallender Initiatoren oder durch Polymerisation mit metallocenhaltigen Katalysatoren erhältlich (EP-A 45 977, US-A 4 857 613, US-A 5 100 978, EP-A 323 716). Die Polymerisation kann dabei sowohl in der Gasphase, als auch in einer Suspension oder in Lösung vorgenommen werden. Derartige Polyolefine sind u.a. unter den Handelsnamen NOVOLEN® und LUPOLEN® von der BASF Aktiengesellschaft erhältlich.

Derartige Polymerisate weisen im allgemeinen mittlere Molmassen (Gewichtsmittel) von 100.000 bis 1.000.000 und Schmelzflußindices von 0,1 bis 100 g/10 min., vorzugsweise von 0,2 bis 80 g/10 min. auf, jeweils gemessen nach DIN 53 735 bei 230°C und 2,16 kg. Der Schmelzflußindex entspricht dabei der Menge an Polymerisat, die innerhalb von 10 Minuten aus der nach DIN 53 735 genormten Prüfvorrichtungen bei einer Temperatur von 230°C und unter einem Gewicht von 2,16 kg ausgepreßt wird.

Homo- und Copolymerisate des Isobutylens werden insbesondere durch kontinuierliche kationische Polymerisation nach dem sogenannten Bandverfahren hergestellt (Ullmans Encyklopädie der Technischen Chemie, Band 19, Seite 220, 4. Auflage, Verlag Chemie GmbH, Weinheim [1980]. Dabei wird das Isobutylen in reinem getrocknetem, flüssigem Ethylen auf einem schwach geneigten, endlosen Stahlband kationisch polymerisiert. Die auf diese Weise erhältlichen Polyisobutylene weisen viskositätsmittlere Molmassen ($\overline{M}_v$) von $0,1 \cdot 10^6$ bis $10 \cdot 10^6$ auf. Sie sind unter dem Handelsnamen OPPANOL® von der BASF Aktiengesellschaft erhältlich.

Mit Hilfe des erfindungsgemäßen Verfahrens erhält man niedermolekulare Polyolefinwachse mit Molmassen $\overline{M}_w$ (Gewichtsmittel) von 1.500 bis 60.000, insbesondere von 3.000 bis 40.000, die auch als Wachse bezeichnet werden. Das erfindungsgemäße Verfahren eignet sich insbesondere zur Herstellung von niedermolekularen Propylenhomopolymerisaten und niedermolekularen Propylencopolymerisaten, die sich gegenüber den aus dem Stand der Technik bekannten niedermolekularen Polyolefinen u.a. durch eine deutlich verringerte Gelbfärbung und durch stark verringerte Anteile an Nebenprodukten, insbesondere von Rußen, auszeichnen. Unter niedermolekularen Propylenhomopolymerisaten sollen dabei sowohl isotaktische als auch syndiotaktische und ataktische Propylenhomopolymerisate verstanden werden. Geeignete niedermolekulare Propylencopolymerisate sind insbesondere sogenannte statistisch verteilte Propylencopolymerisate mit untergeordneten Anteilen anderer $\alpha$-Olefine, beispielsweise von Ethylen und/oder But-1-en.

Das erfindungsgemäße Verfahren zeichnet sich vor allem durch eine gute Steuerbarkeit aus. Es ist verfahrenstechnisch gegenüber den bisher bekannten Abbauverfahren weniger aufwendig, da nachgeschaltete Abbauapparaturen und Verweilzeitaggregate nicht eingesetzt werden müssen. Durch die intensive Durchmischung im Zweiwellenextruder und die Druckveränderungen wird darüber hinaus eine erhöhte Selbstreinigung erzielt, die die Belagbildung an der Reaktorwand unterdrückt. Durch diese Maßnahmen wird u.a. erreicht, daß die Polyolefinketten bevorzugt in der Kettenmitte gespalten werden, während bei einer rein thermischen Spaltung die Spaltstellen statistisch verteilt sind.

Besonders überraschend ist bei dem erfindungsgemäßen Verfahren, daß bei einer Erhöhung des Abbaugrades durch Erzeugen eines zusätzlichen Druckes eine Verringerung der Verfärbung erreicht wird. Durch diese Maßnahmen erhält man niedermolekulare Polyolefine, die sich insbesondere durch einen verringerten Anteil von Nebenprodukten auszeichnen. Sie finden u.a. als Wachse in der Kosmetik, im Farbenbereich, im Druckbereich und als Toner Verwendung.

Beispiele

Die Beispiele 1 und 2 sowie das Vergleichsbeispiel wurden in einem Zweiwellenextruder "ZSK 53" der Firma Werner & Pfleiderer durchgeführt. Der Zweiwellenextruder wurde in eine Aufschmelzzone (I) und eine Zone mit homogener Phase (II) aufgeteilt. Der verwendete Zweiwellenextruder besaß ein Langen/Durchmesser-Verhaltnis von 36.

Beispiel 1

In dem oben genannten Zweiwellenextruder wurden pro Stunde 50 kg eines isotaktischen Propylenhomopolymerisats mit einem Schmelzflußindex von 11 g/10 min. bei 230°C und 2,16 kg nach DIN 53 735 (NOVOLEN® 1100 N der Firma BASF Aktiengesellschaft) bei 420°C und 250 Umdrehungen pro Minute extrudiert. Die durchschnittliche Verweilzeit betrug dabei 2 Minuten. In der Zone mit homogener Phase (II) wurde ein mittlerer Druck von 22 bar angelegt. In den Zweiwellenextruder wurden in Form von mechanischer Energie 6,5 kW (37,1 % der Gesamtenergie) und in Form von thermischer Energie durch Beheizung 11 kW eingebracht. Durch eine Steuervorrichtung wurde der Druck alle 5 Minuten um 2 bar periodisch verändert.

Das daraus erhaltene Abbauprodukt wurde nach Verlassen des Extruders auf 250°C abgekühlt, entgast und durch Pastillieren konfektioniert.

Das auf diese Weise erhaltene Polypropylenwachs wies eine Molmasse $\overline{M}_w$ von 14000, eine Molmassenverteilung Q von 2,2 und eine Schmelze-Viskosität von 104 cst. (bestimmt nach DIN 51 562) auf. Die Gelbfärbung (Yellowness-Index, nach ASTM D 1925, gemessen an Pastillen) betrug 2,9.

Beispiel 2

Das Beispiel 1 wurde unter identischen Bedingungen wiederholt, wobei die Temperatur jetzt 400°C betrug. Der Druck in der Zone (II) wurde auf insgesamt 19 bar eingestellt, die periodische Druckveränderung betrug 0,5 bar.

Das auf diese Weise erhaltene Polypropylenwachs wies eine Molmasse $\overline{M}_w$ von 21000, eine Molmassenverteilung Q von 2,3 und eine Schmelze-Viskosität von 996 cst. (bestimmt nach DIn 51 562) auf. Die Gelbfärbung (Yellowness-Index, nach ASTM D 1925, gemessen an Pastillen) betrug 2,1.

Vergleichsbeispiel

Unter ansonsten gleichen Bedingungen wurde das erfindungsgemäße Beispiel 2 wiederholt, wobei aber in der Zone (II) keine Druckveränderung durchgeführt wurde, so daß der Druck dort konstant bei 19 bar lag.

Das auf diese Weise erhaltene Polypropylenwachs wies eine Molmasse Mw von 21500, eine Molmassenverteilung Q von 2,3 und eine Schmelze-Viskosität von 1004 cst (bestimmt nach DIN 51 562) auf. Die Gelbfärbung (Yellowness-Index nach ASTM D 1925) betrug 2,9.

Weiterhin wurde die Produktqualitat der bei den erfindungsgemaßen Beispielen 1 und 2 und bei dem Vergleichsbeispiel erhaltenen Polypropylenwachse visuell an gegossenen Plättchen einer Größe von 5 cm x 5 cm x 0,3 cm im Hinblick auf Schwarzfärbungen durch Rußpartikel durchgeführt. Die Bewertung erfolgte nach einem abnehmenden Notensystem, wobei 1 für besonders reine und 6 für besonders mit Rußpartikeln verunreinigte Polypropylenwachse steht. Die Proben wurden nach einer Extruderlaufzeit von 1, 5, 12, 24 und 48 Stunden bestimmt. In der nachfolgenden Tabelle sind die jeweiligen Ergebnisse aufgeführt.

Tabelle

| Beispiel 1 | | | | | |
|---|---|---|---|---|---|
| Reaktorlaufzeit [Stunden] | 1 | 5 | 12 | 24 | 48 |
| Zahl der Rußpartikel [Note] | 2 | 2 | 2 | 2 | 2 |
| Beispiel 2 | | | | | |
| Reaktorlaufzeit [Stunden] | 1 | 5 | 12 | 24 | 48 |
| Zahl der Rußpartikel [Note] | 1-2 | 2 | 2 | 2 | 2 |
| Vergleichsbeispiel | | | | | |
| Reaktorlaufzeit [Stunden] | 1 | 5 | 12 | 24 | 48 |
| Zahl der Rußpartikel [Note] | 2 | 2 | 2-3 | 3 | 4 |

Aus der Tabelle wird ersichtlich, daß das erfindungsgemäße Verfahren insbesondere bei längeren Extruderlaufzeiten zu Polypropylenwachsen mit einem deutlich verringerten Anteil an Rußpartikeln führt, was durch einen verbesserten visuellen Eindruck (bessere Noten) zum Ausdruck kommt.

**Patentansprüche**

1.  Verfahren zum thermomechanischen Abbau von Polyolefinen in einem Zweiwellenextruder ohne nachgeschalteter Abbauapparatur, bei Temperaturen von 300 bis 550°C, Drücken von 1 bis 100 bar und mittleren Verweilzeiten des Reaktionsgemisches von 0,5 bis 10 Minuten, dadurch gekennzeichnet, daß der Druck im Zweiwellenextruder periodisch oder aperiodisch mit einer Schwankungsbreite von 0,5 bis 30 bar verändert wird.

2.  Verfahren nach Anspruch 1, dadurch gekennzeichnet, daß der Druck im Zweiwellenextruder periodisch oder aperiodisch mit einer Schwankungsbreite von 0,5 bis 10 bar verändert wird.

3. Verfahren nach einem der Ansprüche 1 oder 2, dadurch gekennzeichnet, daß der Abstand zwischen zwei Druckstößen im Bereich von 1 bis 2000 Sekunden liegt.

4. Verfahren nach einem der Ansprüche 1 bis 3, dadurch gekennzeichnet, daß der thermomechanische Abbau bei Temperaturen von 350 bis 500°C vorgenommen wird.

5. Verfahren nach einem der Ansprüche 1 bis 4, dadurch gekennzeichnet, daß der thermomechanische Abbau bei mittleren Verweilzeiten des Reaktionsgemisches von 0,5 bis 6 Minuten durchgeführt wird.

6. Verfahren nach einem der Ansprüche 1 bis 5, dadurch gekennzeichnet, daß der Zweiwellenextruder in wenigstens zwei Zonen, einer Aufschmelzzone (I) und einer Zone mit homogener Phase (II) aufgeteilt ist, wobei in der zweiten Zone (II) noch ein zusätzlicher Druck von 1 bis 50 bar erzeugt wird.

7. Verfahren nach einem der Ansprüche 1 bis 6, dadurch gekennzeichnet, daß 15 bis 70 % der zum Abbau der Polyolefine benötigten Energie in Form von mechanischer Energie in den Zweiwellenextruder eingebracht werden.

8. Verfahren nach den Ansprüchen 1 bis 7, dadurch gekennzeichnet, daß man es zum Abbau von Propylenhomopolymerisaten und von Propylencopolymerisaten verwendet.

## Claims

1. A process for the thermomechanical degradation of polyolefins in a twin-screw extruder without a downstream degradation apparatus, at from 300 to 550°C and from 1 to 100 bar and in average residence times of the reaction mixture of from 0.5 to 10 minutes, wherein the pressure in the twin-screw extruder is changed periodically or aperiodically with a fluctuation of from 0.5 to 30 bar.

2. A process as claimed in claim 1, wherein the pressure in the twin-screw extruder is changed periodically or aperiodically with a fluctuation of from 0.5 to 10 bar.

3. A process as claimed in either of claims 1 and 2, wherein the interval between two pressure surges is from 1 to 2000 seconds.

4. A process as claimed in any of claims 1 to 3, wherein the thermomechanical degradation is carried out at from 350 to 500°C.

5. A process as claimed in any of claims 1 to 4, wherein the thermomechanical degradation is carried out in average residence times of the reaction mixture of from 0.5 to 6 minutes.

6. A process as claimed in any of claims 1 to 5, wherein the twin-screw extruder is divided into at least two zones, a melting zone (I) and a zone containing a homogeneous phase (II), an additional pressure of from 1 to 50 bar furthermore being generated in the second zone (II).

7. A process as claimed in any of claims 1 to 6, wherein from 15 to 70 % of the energy required for the degradation of the polyolefins are introduced into the twin-screw extruder in the form of mechanical energy.

8. A process as claimed in any of claims 1 to 7, which is used for the degradation of propylene homopolymers and of propylene copolymers.

## Revendications

1. Procédé de dégradation thermomécanique de polyoléfines dans une extrudeuse à deux vis sans appareillage de dégradation en aval, à des températures de 300 à 550°C, sous des pressions de 1 à 100 bars et avec des durées de séjour moyennes du mélange réactionnel de 0,5 à 10 minutes, caractérisé en ce que l'on modifie périodiquement ou non périodiquement la pression dans l'extrudeuse à deux vis avec une plage de fluctuation de 0,5 à 30 bars.

**2.** Procédé suivant la revendication 1, caractérisé en ce que l'on modifie périodiquement ou non périodiquement la pression dans l'extrudeuse à deux vis avec une plage de fluctuation de 0,5 à 10 bars.

**3.** Procédé suivant la revendication 1 ou 2, caractérisé en ce que la distance entre deux coups de pression varie dans la gamme de 1 à 2000 secondes.

**4.** Procédé suivant l'une quelconque des revendications 1 à 3, caractérisé en ce que l'on entreprend la dégradation thermomécamique à des températures de 350 à 500°C.

**5.** Procédé suivant l'une quelconque des revendications 1 à 4, caractérisé en ce que l'on entreprend la dégradation thermomécanique avec des durées de séjour moyennes du mélange réactionnel qui varient de 0,5 à 6 minutes.

**6.** Procédé suivant l'une quelconque des revendications 1 à 5, caractérisé en ce que l'extrudeuse à deux vis est subdivisée en au moins deux zone, à savoir une zone de fusion (I) et une zone à phase homogène (II), où dans la seconde zone (II) s'établit encore une pression supplémentaire de 1 à 50 bars.

**7.** Procédé suivant l'une quelconque des revendications 1 à 6, caractérisé en ce que de 15 à 70% de l'énergie nécessaire à la dégradation des polyoléfines sont introduits sous forme d'énergie mécanique dans l'extrudeuse à deux vis.

**8.** Procédé suivant l'une quelconque des revendications 1 à 7, caractérisé en ce qu'on le met en oeuvre en vue de la dégradation d'homopolymères du propylène et de copolymères du propylène.